# EUROPEAN PATENT APPLICATION

(11) **EP 3 095 507 A1**
(43) Date of publication of application: **23.11.2016**
(21) Application number: 15168280.4
(22) Date of filing: 19.05.2015
(51) Int. Cl.: B01D 63/02, C02F 3/20

(54) **MEMBRANE CONNECTOR TO PROMOTE MASS TRANSFER IN A MEMBRANE AERATED BIOFILM REACTOR (MABR)**

(71) Applicant: OxyMem Limited, Athlone Co. Westmeath (IE)
(72) Inventor: The designation of the inventor has not yet been filed
(74) Representative: Purdy, Hugh Barry

(57) **Abstract**

A membrane fibre bunch for use in a membrane-aerated biofilm reactor (MABR), the MABR having a housing, the membrane fibre bunch comprising: a group of membrane fibres arranged as a bunch of vertical or horizontal membrane fibres attached at either end to an gas supply manifold within the housing, with each fibre having a lumen containing a gas phase; and a means for connecting the group of membrane fibres so that the gas can flow within the lumen of the membrane; wherein the group of membrane fibres are maintained in position by a connector or overmold, the connector or overmold configured to maintain the group of membrane fibres in a spaced-apart configuration from one another.

## Description

### Field of the Invention

The invention relates to membrane aerated biofilm reactors (MABRs). In particular, the invention relates to a device for use in a MABR, the device capable of increasing the mass transfer between the wastewater and the membrane attached biofilm.

### Background to the Invention

As the world's population continues to grow, and more and more people move to live in urban environments, there are increased demands being placed on the wastewater treatment infrastructure. Both municipal and industrial wastewater treatment plants are having to achieve higher and higher water quality levels to meet regulatory standards, which at the same time must be achieved in smaller spaces, utilising less resources. This is driving innovation in wastewater treatment technology. One such technology which can achieve these increased treatment rates while at the same time utilising less resources is the Membrane Aerated Biofilm Reactor (MABR). The MABR utilises gas permeable membranes to provide oxygen to the pollutant degrading bacteria which grow attached to the membrane surface. The MABR combines the low energy requirements of permeable membrane aeration with the key advantages of biofilm-based processes such as the high volumetric reaction rate that can be attained due the high specific biomass concentration. Biofilm based treatment systems have become more common in recent years. These systems generally place moving media into reactor tanks, the biofilm attaches to the media and both move about the tank freely with the liquid as it is mixed. Such Biofilm Reactors are described in US Patent No. 7189323.

Two issues remain with biofilm-based systems: 1) a lot of energy is required to provide both the oxygen necessary for the biofilm and the energy required to mix the water and the media while in these systems; 2) the depth to which the biofilm is active is very thin due the dissolved oxygen being consumed in the outer layers of the biofilm and results in the inner layers of the biofilm not being aerobically active. Both of these issues are overcome by the MABR.

The MABR has several advantages over conventional biofilm technologies:
(1) comparatively high volumetric carbon oxygen demand (COD) removal rates are achievable especially if pure oxygen is fully exploited and if biofilm thickness-control measures are in place.
(2) bubbleless aeration offers the potential for significantly higher oxygen utilization efficiencies with consequent energy savings. In addition, reduced air stripping during the biotreatment of volatile organic compounds is possible.
(3) simultaneous nitrification, denitrification and COD removal can be achieved at comparatively higher rates due to the unique microbial population stratification.
(4) specialist degrading microorganisms, such as ammonia oxidizing bacteria, tend to be preferentially grow adjacent to the biofilm-membrane interface thereby being protected from biofilm erosion.

The MABR technology is beginning to make the transition from research scale to full scale deployments. The problem being experienced by upscaling the size of the MABRs is that contact (mixing) between the pollutant-containing wastewater and the biofilm attached to the support media is inadequate. Therefore additional mixing and enhanced mass transfer is essential but this must be performed without a significant increase in energy as this would erode the major commercial advantage of the MABR.

It is an objective of the present invention to overcome at least one of the above-mentioned problems.

### Summary of the Invention

In the MABR, the biofilm is naturally immobilized on an oxygen permeable membrane. Oxygen diffuses through the membrane into the biofilm where oxidation of pollutants, supplied at the biofilm-liquid interface, takes place. The oxygen supply rate is controlled by the intra-membrane oxygen partial pressure (a process parameter) and membrane surface area (a design parameter). The object of the present application is to maximise the mass transfer of oxygen from the gas within the membranes of the MABR to the biofilm on the membrane surface. It is also an object of the present invention to maximise the rate of mass transfer of the pollutants from the wastewater into the pollutant degrading biofilm by exposing more of membrane fibre surface to the polluted wastewater. These combined advantages of the invention increase the rate of wastewater treatment of the MABR. The arrangement of the membrane fibres into groups with unique and innovative cross sectional shapes avoids, prevents, and/or minimises the occurrence of dead zones or nonreactive zones or stagnant zones within the membrane bunch and in between the membrane bunches when they are arranged into cassettes and modules within the reactor. The inclusion of the unique membrane arrangement not only increases the exposed surface area of the membranes but, due to the non-uniform arrangement of these membrane groups, turbulence is created in the liquid flow, thus further improving the mass transfer from the membranes and to the biofilm.

To ensure the MABR can become an effective full-scale technology for wastewater treatment, there is a critical need to ensure that the reactor is well mixed and that there is effective contact between as much of the surface area of the membrane-attached pollutant-degrading biofilm and the polluted wastewater to be treated. The Applicants have provided a solution for maximising the contact between the wastewater and the pollutant degrading biofilm in a MABR treatment system or tank. The solution creates increased mixing due to turbulence within the system and without any additional energy requirement. The system thereby maintains the low energy demand of the MABR for biological wastewater treatment.

This is done in a way that is easy to manufacture and assemble, and does not require the addition of a weave or wrap.

According to the present invention there is provided, as set out in the appended claims, a membrane-aerated biofilm reactor (MABR) of the type comprising: a housing having an upper air manifold and a lower air manifold; at least one array of membranes disposed within the housing and arranged in between and extending from the upper air manifold to the lower air manifold, each membrane array consisting of a bunch of membrane fibres defining a lumen containing a gas phase; and a means for connecting the at least one bunch of membranes to the upper and lower manifold so that the gas can flow within the lumen of the membrane; wherein the bunch of membrane fibres are maintained in position by a semi-rigid or rigid connector, the connector configured to maintain the membrane fibres in a spaced-apart configuration from one another.

According to the present invention, there is provided, as set out in the appended claims, a membrane fibre bunch for use in a membrane-aerated biofilm reactor (MABR), the MABR having a housing, the membrane fibre bunch comprising: a group of membrane fibres arranged together as a bunch of vertical or horizontal membrane fibres, with each fibre having a lumen containing a gas phase; and a means for connecting the bunch of membrane fibres so that the gas can flow within the lumen of the membrane; wherein the bunch of membrane fibres are arranged together by a connector or overmold, the connector or overmold configured to arrange and maintain the bunch of membrane fibres in a spaced-apart configuration from one another.

In one embodiment, the bunch of membrane fibres are bound together by a semi-rigid or rigid connector. Preferably, the connector or overmold has a preformed profile shape.

Preferably, the connector and overmold are neither circular nor cylindrical in profile. A unique profile may be created by means of placing the bunch of membrane fibres into (i) a pre-formed connector or (ii) by means of holding the bunch of membrane fibres in the unique profile and fixing them in this configuration by means of placing an adhesive over the fibers so as to maintain the fibres in this configuration or by moulding the connector around the fibres to keep them in place (an overmold). In one embodiment, the connector or overmold has a profile shape selected from the group comprising an ellipse, a three-pointed star, a four-pointed star, a five-pointed star, a cross-shape, a chevron, or any shape suitable to provide a more widely distributed and mixed flow across the entire bunch of membrane fibres.

In one embodiment, the bunch of membrane fibres are attached at either end to an air supply manifold.

In one embodiment, a plurality of membrane fibre bunches are placed in sequence to form an array. Ideally, each membrane fibre bunch in the array share a common air supply manifold.

In one embodiment, the array comprises a plurality of membrane fibre bunches placed side by side, which forms a cassette. Preferably, several cassettes are placed together to form a module, which when placed within the housing, substantially occupies the space within the housing of the MABR. Ideally, each membrane fibre bunch in the array is attached to an upper and lower air manifold to form the cassette. The cassette is then placed into the housing and fixed in position to form a MABR. The positioning of the membrane fibre bunches in the array provides a well distributed and mixed flow throughout all of the membrane fibre bunches in the array (also known as membrane cassettes).

In one embodiment, the profile of the group of membrane fibres in the membrane bunch may be narrow and extended. The narrowing and extending of the profile of the membrane fibres in the membrane bunch reduces the distance between (all of) the fibres and any bulk liquid to be treated. This permits a more widely distributed and mixed flow across the entire bunch of membrane fibres.

In one embodiment, the connector is configured to be attached to an air supply manifold. The connector can be attached to the air supply manifold via a fitting, or adhered directly to the manifold with any suitable adhesive known to the skilled person such as glue, epoxy resin and the like.

The extension of the membrane fibres into the liquid to be treated, and away from the central axis of the bunch of vertical membrane fibres, reduces the distance between the central membrane fibres and the liquid, and maximises the exposed surface area of the membrane fibres. The arrangement of membrane fibres in the bunch provides a narrow profile, reducing the distance between all of the membrane fibres and any liquid in the housing to be treated. The configuration of the membrane fibres in the bunch provides a higher effective rate of oxygen transfer in the reactor.

In the specification, the term "Membrane Aerated Biofilm Reactor (MABR)" should be understood to mean a membrane supported biofilm reactor (MSBR) or Membrane Biofilm Reactor (MBfR) for treating water and or wastewater liquids to remove carbonaceous pollutant removal, nitrify/denitrify the pollutants, and/or perform xenobiotic biotreatment of the wastewater constituents, and employing an air/oxygen/hydrogen (gas) permeable membrane (often a hollow fibre membrane) that provides an interface between the fluid to be treated (fluid phase) and an air/oxygen/hydrogen supply (gas phase). Soluble organic compounds in the liquid are supplied to the biofilm from the biofilm-liquid interface, whereas air/oxygen/hydrogen/carbon dioxide or other gas supply to the biofilm is from the biofilm-membrane interface (by diffusing through the membrane). Typically, a biofilm consisting of a heterogeneous population of bacteria (micro-organisims) (generally including nitrifying, denitrifying, and heterotrophic, bacteria) grows on the fluid phase side of the membrane. MABRs can achieve bubble-less aeration and high oxygen utilization efficiency (up to 100%) and the biofilm can be separated into aerobic/anoxic/anaerobic zones to simultaneously achieve removal of carbonaceous organic pollutants, as well as nitrification and denitrification in a single biofilm. An example of MABRs of the type comprising a lumen containing a gas phase, a liquid phase, and a gas permeable membrane providing an interface between the gas and liquid phases are described by European Patent No. 2 361 367 (University College Dublin).

In the specification, the term "bunch of membrane fibres" should be understood to mean a plurality of membrane fibres arranged together as a bunch. The number of membrane fibres in a single bunch can be any number, for example from 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36,37,38,39,40,41,42,43,44,45,46,47,48,49,50,51,52,53,54,55,56,57,58,59, 60, 61, 62, 63, 64, 65, 66, 67, 68, 69, 70, 71, 72, 73, 74, 75, 76,. 77, 78, 79, 80, 81, 82, 83, 84, 85, 86, 87, 88, 89, 90, 91, 92, 93, 94, 95, 96, 97, 98, 99, or 100 membrane fibres. Typically, the fibres in a bunch are parallel and ideally co-extensive.

In the specification, the term "membrane cassette" should be understood to mean a plurality of bunches of membrane fibres arranged side-by-side and typically attached to an upper and lower air manifold. The number of bunches of membrane fibres in a cassette can be any number, for example from 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76,. 77, 78, 79, 80, 81, 82, 83, 84, 85, 86, 87, 88, 89, 90, 91, 92, 93, 94, 95, 96, 97, 98, 99, or 100 bunches of membrane fibres.

In the specification, the term "chevron profiles" should be understood to mean a group of hollow membrane fibres which are arranged together, so that when observed from a horizontal plane, they form the shape of a chevron.

In the specification, the term "housing" should be understood to mean a holding vessel, such as a tank, within which is placed the MABR. The term "housing" and "tank" may be used interchangeably.

In the specification, the terms "upper manifold" and "lower manifold" should be understood to mean air manifolds connected to either ends of the hollow fibre membranes. The manifolds provide a means to supply and remove the gas to/from the lumen of the membranes. If the fibres are placed in the vertical orientation, the manifolds are then in the upper and lower portions of the MABR housing.

### Brief Description of the Drawings

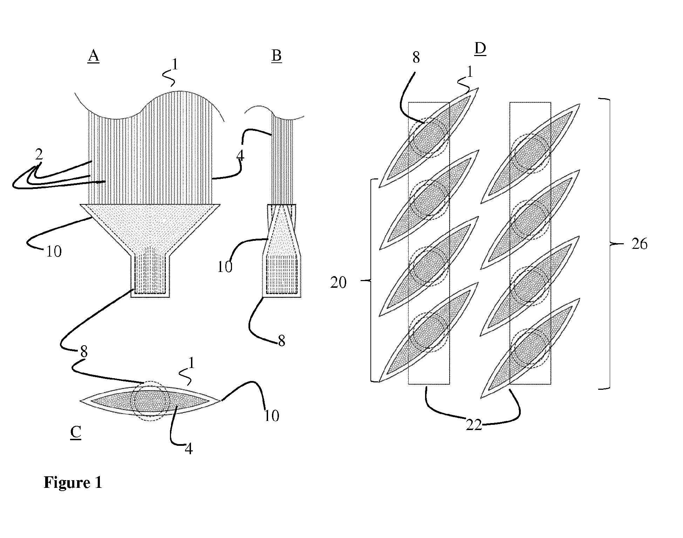

The invention will be more clearly understood from the following description of an embodiment thereof, given by way of example only, with reference to the accompanying drawings, in which:-
**Figure 1** illustrates (A) an elevation view, (B) an side view and (C) a plan view of one embodiment of a bunch of membrane fibres arranged by a semi-rigid/rigid connector or overmold to form an array with a particular profile for use in a membrane aerated biofilm reactor (MABR) device. Figure 1(D) illustrates a plan view of two adjoining arrays of the claimed invention. The bunches of membrane fibres are arranged into a single array, which can be placed side-by-side with other arrays to form a cassette and distributed flow.

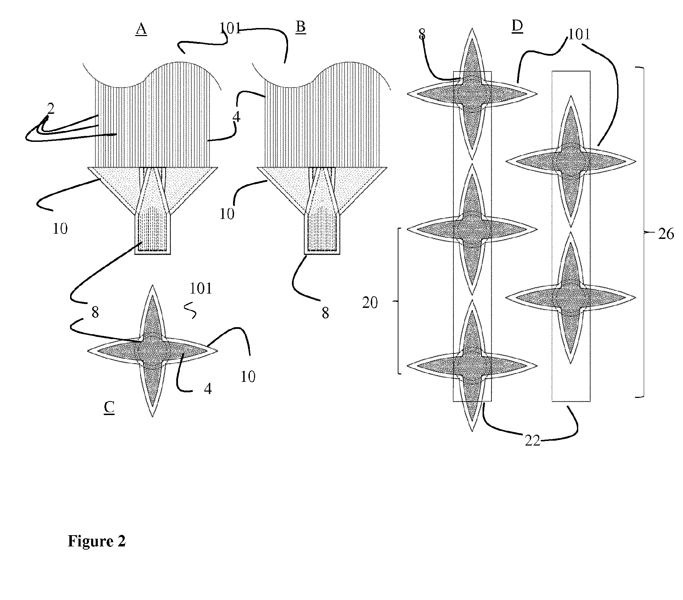

**Figure 2** illustrates (A) an elevation view, (B) an side view and (C) a plan view of one embodiment of a bunch of fibres arranged by a semi-rigid/rigid connector or overmold to form an array with a particular profile for use in the membrane aerated biofilm reactor (MABR) device. Figure 2(D) illustrates a plan view two adjoining arrays of the claimed invention. The bunches of membrane fibres are arranged into a single array, which can be placed side-by-side with other arrays to form a cassette and distributed flow.

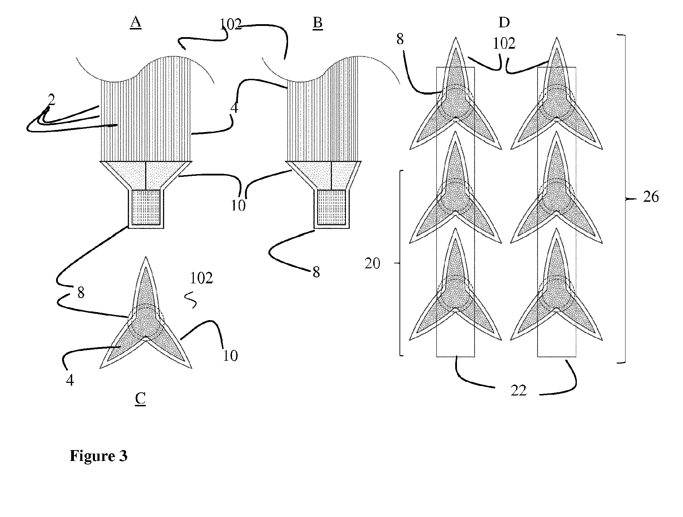

**Figure 3** illustrates (A) an elevation view, (B) an side view and (C) a plan view of one embodiment of a bunch of fibres arranged by a semi-rigid/rigid connector or overmold to form an array with a particular profile for use in the membrane aerated biofilm reactor (MABR) device. Figure 3(D) illustrates a plan view two adjoining arrays of the claimed invention. The bunches of membrane fibres are arranged into a single array, which can be placed side-by-side with other arrays to form a cassette and distributed flow.

### Detailed Description of the Drawings

The invention relates to a device for treating wastewater liquids, the device is suitable for use a membrane supported biofilm reactor (MSBR) or a Membrane Aerated Biofilm Reactor (MABR). The device comprising a plurality of membranes arranged into a bunch having a specific profile/configuration, with each membrane consisting of a lumen containing a gas phase, a liquid phase, and a gas permeable membrane providing an interface between the gas and liquid phases; and a means for holding the membranes in a defined configuration to maintain a high exposure of the membrane's exposed surface area and ensure that the membranes are adequately dispersed in the wastewater

The membrane bunches which are held in the defined configuration are then placed in an array with a gas manifold at either end of the membrane bunches allowing for air/oxygen/hydrogen/methane/carbon dioxide or other gas to be supplied to the membrane lumen and removed from the membrane lumen at the opposite side. The array of bunches along with the manifold (cassette) are then arranged in the housing to maximise the contact between the membranes and the liquid contained in the housing.

Referring now to the figures, where **Figure 1** illustrates a general embodiment of the device of the present invention. Specifically, **Figures 1A** to **1C** illustrate (A) an elevation view, (B) an side view and (C) a plan view of a typical device of the present invention, and is generally referred to by reference numeral 1. The device 1 comprises a group of membrane fibres 2 arranged together as a bunch of vertical membrane fibres 4 attached at either end to an air supply manifold 8. A biofilm is grown and accumulates upon the densely packed bunch of membrane fibres 4. Each membrane fibre 2 has a lumen containing a gas phase. The bunch of membrane fibres 4 are connected and arranged together by a connector or overmold 10. The connector or overmold 10 is configured to arrange and maintain the bunch of membrane fibres 4 in a spaced-apart configuration from one another. This provides the device 1 with a group of membrane fibres 2 that allows the gas to flow within the lumen of each of the membranes 2. The connector or overmold 10 also imparts a unique profile on the bunch of membranes 4, such as the elliptical profile illustrated in Figure 1. As illustrated in **Figure 1** (and **Figures 2** **and** **3**), the connector or overmmold 10 is configured to attach to the air supply manifold 8.

When a plurality of a bunch of membrane fibres 4 are arranged by a semi-rigid/rigid connector or overmold 10 and placed in sequence, an array 20 is formed. The array 20 now comprises a plurality of membrane fibre bunches 4 with a particular profile imparted to them by the connector or overmold 10. Each bunch of membrane fibres 4 in the array 20 are attached to an upper and lower air manifold 22 to form a cassette 26. Figure 1(D) illustrates two adjoining cassettes 26 comprising the arrays 20 of the claimed invention.

The positioning of the membrane fibre bunches 4 in the array 20 provides a well distributed and mixed flow throughout all of the membrane fibre bunches 4 in the array 20. The cassette 26 can be placed and fixed within a housing of a membrane aerated biofilm reactor (MABR) device to form a module.

Referring to **Figures 2** and **3**, there is illustrated additional embodiments of the device in which parts or steps described with reference to the previous embodiments are assigned the same numerals. In the embodiment, the device 200, 300 comprise a group of membrane fibres 2 arranged together as a bunch of vertical membrane fibres 4 attached at either end to an air supply manifold 8. A biofilm is grown and accumulates upon the densely packed bunch of membrane fibres 4. Each membrane fibre 2 has a lumen containing a gas phase. The bunch of membrane fibres 4 are connected and arranged together by a connector or overmold 101, 102. The connector or overmold 101, 102 is configured to arrange and maintain the bunch of membrane fibres 4 in a spaced-apart configuration from one another. This provides the device 200, 300 with a group of membrane fibres 2 that allows the gas to flow within the lumen of each of the membranes 2. The connector or overmold 101, 102 also imparts a unique profile on the bunch of membranes 4, such as the four-pointed star profile illustrated in Figure 2 and the three-pointed star profile illustrated in Figure 3. It should be understood that the profile of the device 1, 101, 102 can be any profile that provides a more widely distributed and mixed flow across the entire bunch of membrane fibres 4, such as a five-pointed star, a cross-shape, a chevron, and the like.

From a biocatalytic point of view, the more membranes and biofilm in a wastewater treatment housing the better. However, above a certain limit, the accumulation of biofilm can cause severe problems with liquid flow distribution. Therefore, an effective membrane distribution must be maintained. Commercially, the membrane packing density must be increased to provide the most gas transferring membranes per unit of reactor volume. Many of the laboratory scale studies reported to-date in the literature were operated with low membrane packing densities. This current invention describes a device 1, 101, 102 to allow for better distribution of high packing densities of membrane aeration fibres within a membrane aerated biofilm reactor. The current invention also results in effective contact of the liquid waste with all of the membranes 2 in the reactor.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A membrane fibre bunch for use in a membrane-aerated biofilm reactor (MABR), the MABR having a housing, the membrane fibre bunch comprising: a group of membrane fibres arranged in a bunch, with each fibre having a lumen containing a gas phase; and a means for connecting the group of membrane fibres to a manifold so that in use gas can flow within the lumen of the membrane; wherein the membrane fibres in the bunch are arranged together by a connector or overmold, the connector or overmold configured to arrange and maintain the membrane fibres in the bunch in a spaced-apart configuration from one another.

2. A membrane fibre bunch as claimed in Claim 1, wherein the connector is semi-rigid or rigid.

3. A membrane fibre bunch as claimed in Claim 1 or Claim 2, wherein the connector or overmold has a preformed profile shape.

4. A membrane fibre bunch according to any one of Claims 1 to 3, wherein the connector or overmold has a profile shape selected from the group comprising an ellipse, a three-pointed star, a four-pointed star, a five-pointed star, a cross-shape, a chevron or any other shape.

5. A membrane fibre bunch according to any one of the preceding claims, wherein the membrane fibre bunch is attached at either end to an air supply manifold.

6. A membrane fibre bunch according to any one of the preceding claims, wherein the group of membrane fibres of the bunch extend into the housing and away from a central axis of the group of membrane fibres, and maximises an exposed surface area of the membrane fibres.

7. A membrane fibre bunch according to any one of the preceding claims, wherein the connector is configured to be attached to an air supply manifold.

8. A membrane fibre bunch according to Claim 9, wherein the connector is attached to the air supply manifold by a push-fit type fitting or affixed directly thereto.

9. A membrane fibre bunch according to any one of the preceding claims, wherein the membrane fibre bunch is disposed within the housing in the form of a cassette.

10. An array comprising a plurality of membrane bunch fibres arranged in sequence.

11. An array according to Claim 10 in which the plurality of membrane bunch fibres are in fluid communication with each other.

12. A cassette comprising a plurality of membrane fibre bunches of any of Claims 1 to 9 or a plurality of arrays of Claims 10 or 11 arranged side-by-side and attached to an upper and lower air manifold.

13. A membrane-aerated biofilm reactor (MABR) of the type comprising a housing and at least one membrane fibre bunch as claimed in any of Claims 1 to 9.

14. A membrane-aerated biofilm reactor (MABR) comprising a housing and at least one array according to Claim 10 or 11.

15. A membrane-aerated biofilm reactor (MABR) comprising a housing and at least one cassette according to Claim 12.
